# EUROPÄISCHE PATENTSCHRIFT

(11) **EP 1 032 572 B1**
(45) Veröffentlichungstag und Bekanntmachung des Hinweises auf die Patenterteilung: **11.12.2002**
(21) Anmeldenummer: 98965668.1
(22) Anmeldetag: 12.11.1998
(51) Int. Cl.: C07D 407/00, C07D 407/06, C07D 309/36, A61K 31/365

(54) **PYRANONE, VERFAHREN ZU IHRER HERSTELLUNG UND VERWENDUNG**
PYRANONES, METHOD FOR THE PRODUCTION AND USE THEREOF
PYRANONES, LEUR PROCEDE DE PRODUCTION ET LEUR UTILISATION

(30) Priorität: 14.11.1997 DE 19750623
(43) Veröffentlichungstag der Anmeldung: 06.09.2000
(73) Patentinhaber: Dr. Willmar Schwabe GmbH & Co., 76227 Karlsruhe (DE)
(72) Erfinder: CHATTERJEE, Shyam, Sunder, D-76139 Karlsruhe (DE); HAUER, Hermann, D-76228 Karlsruhe (DE)
(74) Vertreter: Bunke, Holger, Dr.rer.nat. Dipl.-Chem.
(86) Internationale Anmeldenummer: EP9807241
(87) Internationale Veröffentlichungsnummer: WO99025716

(56) Entgegenhaltungen:
- DE-B- 1 277 263
- DE-B- 1 279 683
- H.J. MEYER ET AL.: "UNTERSUCHUNGEN ÜBER BEZIEHUNGEN ZWISCHEN MOLEKULARSTRUKTUR U. PHARMAKOLOGISCHER WIRKUNG C6 ARYLSUBSTIT. 4-METHOXY-ALFA-PYRONE" ARZNEIMITTEL FORSCHUNG. DRUG RESEARCH., Bd. 19, 1969, Seiten 617-623, XP002101580 AULENDORF DE in der Anmeldung erwähnt
- SIB S. TETRAHEDRON Bd. 31, 1975, Seiten 2229 - 2235

## Beschreibung

Die Erfindung betrifft Pyranone, deren Grundstruktur sich von Laktonen, die in der Pflanze *Piper methysticum* vorkommen, ableiten läßt, Verfahren zur Herstellung dieser Verbindungen, einschließlich der dabei entstehenden Zwischenprodukte, sowie Arzneimittel, die diese Verbindungen enthalten.

Die Verwendung von Kawa (Piper methysticum)-Extrakten zur Therapie von Angst und ähnlichen psychischen Erkrankungen ist schon lange bekannt. Es ist ebenfalls bekannt, daß die aktive Komponente solcher Extrakte die sogenannten Kawapyrone (Kawain, Dihydrokawain, Methysticin, Dihydromethysticin und Yangonin) sind. Zahlreiche pharmakologische Untersuchungen belegen die verschiedenen biologischen Effekte solcher Reinstoffe sowie ihre krampfhemmenden und neuroprotektiven Eigenschaften [vgl. R. Kretzschmar et al., Arch. Int. Pharmacodyn. Ther. 177, 261ff. (1969) und C. Backhauß et al., Europ. J. Pharmacol. 215, 265ff. (1992)]. Bis jetzt ist aber die praktische therapeutische Anwendung der Kawapyrone als Antiepileptika oder zur Behandlung neurodegenerativer Erkrankungen nicht bekannt geworden. Wir haben festgestellt, daß die bekannten Kawapyrone für die Therapie von Epilepsien und neurodegenerativen Erkrankungen schlecht geeignet sind, weil die Wirkdauer dieser Substanzen nach oraler Gabe sehr kurz und außerdem die orale Bioverfügbarkeit mancher dieser Naturstoffe zu schlecht ist. Darüber hinaus steht der gewerblichen Herstellung entsprechender Arzneimittel hindernd entgegen, daß die Isolierung neuer Naturstoffe aus der Kawawurzel oder anderen Pflanzen sehr aufwendig ist, was die Therapie mit solchen Verbindungen sehr teuer, und damit unwirtschaftlich, werden ließe. Der gewerblichen Verwertung steht femer entgegen, daß die Naturstoffe optisch aktiv sind, wohingegen es sich bei den durch einfachere Synthese hergestellten bisher bekanntgewordenen Derivaten der Kawalaktone um Racemate handelt. Da die Wirkungen der Kawalaktone von ihrer Enantiomerform abhängen, können für Therapiezwecken nur solche Kawalaktone verwendet werden, die stereoselektiv synthetisiert werden. Bis jetzt sind jedoch für diese Stoffklasse keine solchen kommerziell anwendbaren stereo-spezifischen Synthesemethoden bekannt.

Aus DE-A-1 277 263 ist ein Verfahren zur Herstellung von Dihydromethysticin und Dihydrokawain durch katalytische Hydrierung von Methysticin und Kawain bekannt. Dabei wird auf verschiedene medizinische Indikationen dieser Verbindungen hingewiesen: Dihydrokawain wirkt als Antiphlogistikum und Endoanästhetikum, Dihydromethysticin als schlafinduzierendes Mittel und ebenfalls als Endoanästhetikum.

Aus der DE-A-1 279 683 ist ein Verfahren zur Herstellung substituierter α-Pyrone, nämlich von Dehydrokawain und Dehydromethysticin bekannt, bei dem Piperonal oder ein anders substituierter Benzaldehyd mit 4-Methoxy-6-methyl-α-pyron kondensiert wird. Die dadurch erzeugten substituierten α-Pyrone sind pharmakologisch ebenfalls wirksam als Antiphlogistikum, Endoanästhetikum und schlafinduzierendes Mittel.

Der Erfindung liegt deshalb die Aufgabe zugrunde, neue Pyranon-Derivate zu schaffen, die bei oraler Gabe hinreichend stark antikonvulsiv wirksam sind und eine mehrstündige Wirkdauer aufweisen und als Arzneimittelwirkstoffe, insbesondere in der Therapie von Erkrankungen des ZNS, verwendet werden können.

Diese Aufgabe wird durch die Bereitstellung der erfindungsgemäßen Verbindungen und Verfahren sowie durch die Verwendung dieser Verbindungen als antikonvulsiv und antiepileptisch oral wirksame Arzneimittel gelöst.

Gegenstand der Erfindung sind somit:
2H-1-Pyran-2-one der allgemeinen Formel I, 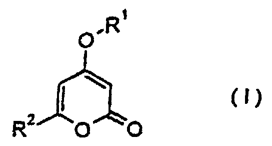 worin
- R¹: einen Alkylrest mit 2 bis 5 C-Atomen, einen Cycloalkylrest mit 4 bis 6 C-Atomen, einen Cycloalkyl-alkylrest mit 4 bis 8 C-Atomen oder einen Alkoxyalkylrest mit insgesamt 3 bis 5 C-Atomen und
- R²: einen am Benzolring in den Positionen 3, 4 und 5 mit R³, R⁴ und R⁵ substituierten 2-Phenylethenylrest
darstellen, wobei unabhängig voneinander
- R³ und R⁵: Methoxy oder Ethoxy und
- R⁴: ein geradkettiger oder verzweigter Alkoxyrest mit 1 bis 5 C-Atomen, ein Alkenyloxyrest mit 3 bis 5 C-Atomen, ein Cycloalkoxyrest mit 4 bis 6 C-Atomen, ein Cycloalkylalkoxyrest mit 4 bis 8 C-Atomen oder einen Alkoxyalkoxyrest mit insgesamt 3 bis 5 C-Atomen sind.

Die erfindungsgemäßen Verbindungen sind neu.

Bekannt ist die Verbindung 4-Methoxy-6-[2-(3,4-methylendioxyphenyl)ethyl]-2H-pyran-2-on (H. J. Meyer und R. Kretzschmar, Arzneim.-Forsch. 19, 617 (1969)) und deren mäßig antikonvulsive Wirkung bei i.v. Gabe. Bekannt sind auch die natürlich vorkommenden Kawa-Laktone Methysticin (E-(+)-6-[2-(1,3-Benzodioxol-5-yl)ethenyl]-5,6-dihydro-4-methoxy-2H-pyran-2-on), Dihydromethysticin ((+)-6-[2-(1,3-Benzodioxol-5-yl)ethyl]-5,6-dihydro-4-methoxy-2H-pyran-2-on), Kawain (E-(+)-5,6-Dihydro-4-methoxy-6-(2-phenylethenyl)-2H-pyran-2-on), Dihydrokawain ((+)-5,6-Dihydro-4-methoxy-6-(2-phenylethyl)-2H-pyran-2-on), Yangonin (E-4-Methoxy-6-[2-(4-methoxyphenyl)ethenyl]-2Hpyran-2-on) und Desmethoxy-yangonin (E-4-Methoxy-6-(2-phenylethenyl)-2H-pyran-2-on), sowie deren antikonvulsive Wirkung bei oraler Gabe (R. Kretzschmar und H. J. Meyer, Arch. Int. Pharmacodyn. Ther. 177, 261 (1969)). Diese Verbindungen sind jedoch im MES-Test nach 3 h kaum noch wirksam.

Im Hinblick auf diesen Stand der Technik war es für den Fachmann überraschend und in keiner Weise vorhersehbar, daß die erfindungsgemäßen Verbindungen der allgemeinen Formel I eine gute antikonvulsive und antiepileptische Wirkung aufweisen, die bei oraler Gabe über mehrere Stunden anhält, und damit vielfach eine längere Wirkungsdauer besitzen als die natürlich vorkommenden Kawalaktone. Ein wichtiger, mit der Erfindung erzielter Vorteil besteht darin, daß auf die aufwendige Isolierung von Naturstoffen verzichtet werden kann und einfache Syntheseverfahren, die keine stereosetektive Verfahrensführung erfordern, angewandt werden können, da die neuen Stoffe achiral sind, d.h. keine Asymmetriezentren besitzen.

Erfindungsgemäß bevorzugte Verbindungen der allgemeinen Formel I sind solche, bei denen
- R¹: einen Alkylrest mit 2 bis 5 C-Atomen, einen Cyclopropylmethylrest oder einen Methoxyethylrest und
- R²: einen am Benzolring in den Positionen 3, 4 und 5 mit R³, R⁴ und R⁵ substituierten 2-Phenylethenylrest
darstellen, wobei unabhängig voneinander
- R³ und R⁵: Methoxy und
- R⁴: ein geradkettiger oder verzweigter Alkoxyrest mit 1 bis 5 C-Atomen, ein Allyloxyrest, ein Cyclopentyloxyrest, ein Cyclopropylmethoxyrest oder ein Methoxyethoxyrest sind.

Die Verbindungen der allgemeinen Formel I, bei denen R² ein am Benzolring in den Positionen 3, 4 und 5 mit R³, R⁴ und R⁵ substituierten 2-Phenylethenylrest ist, werden durch Kondensation des entsprechend mit R¹ substituierten Pyranons der allgemeinen Formel III mit einem in den Positionen 3, 4 und 5 mit R³, R⁴ und R⁵ substituierten Benzaldehyd der allgemeinen Formel IV hergestellt, wobei R³, R⁴, und R⁵ die oben genannten Bedeutungen haben.

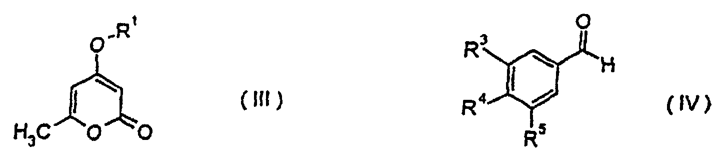

Die Verbindungen der allgemeinen Formel III werden durch Alkylierung von 4-Hydroxy-6-methyl-2H-pyran-2-on mit R¹X erhalten.

Die Verbindungen der allgemeinen Formel IV werden durch O-Alkylierung eines entsprechend mit R³, R⁴ und R⁵ substituierten Benzaldehyds erhalten, bei dem ein oder zwei der Reste R³ bis R⁵ die oben angegebene Bedeutung haben und die verbleibenden Reste R³ bis R⁵ Hydroxy sind.

Die Vorprodukte III sind, soweit sie neu sind, ebenfalls Gegenstand der Erfindung.

Gegenstand der Erfindung sind ferner oral wirksame Arzneimittel, die als Wirkstoff eine oder mehrere der erfindungsgemäßen Verbindungen der allgemeinen Formel I sowie gegebenenfalls zusätzlich pharmakologisch inerte Hilfsstoffe wie zum Beispiel Wasser, pflanzliche Öle, Polyethylenglykole, Glycerinester, Gelatine, Kohlenhydrate wie Lactose oder Stärke, Magnesiumstearat, Talk, Vaseline®, Konservierungsmittel, Netzmittel, Emulgatoren, physiologisch unbedenkliche Salze, Puffersubstanzen, Farbstoffe, Geschmacksstoffe und Aromastoffe enthalten. Die Wahl der Begleitstoffe hängt davon ab, ob die Arzneimittel in Form von Tabletten, Dragees, in flüssiger Form oder in Form von Sprays oral applizierbar sein sollen. Die erfindungsgemäßen Verbindungen können auch im Gemisch mit anderen bekannten Wirkstoffen verabreicht werden. Die orale Wirksamkeit dieser Arzneimittel schließt nicht aus, daß die erfindungsgemäßen Wirkstoffe und Arzneimittel auch bei nicht-oraler Applikation wirksam sind.

In den nachfolgenden Beispielen sind die erfindungsgemäßen Verbindungen, die Verfahren zu ihrer Herstellung sowie die pharmakologischen Versuchsergebnisse näher beschrieben.

Die im nachfolgenden Text verwendeten Abkürzungen bedeuten: TBME = tert-Butyl-Methylether, PE = Petrolether, DMSO = Dimethylsulfoxid und DMF = N,N-Dimethylformamid. Unter "einrotieren" wird das Abziehen von Lösemittel(resten) bis zur Trockne unter Verwendung eines Rotationsverdampfers verstanden.

### I. Beispiele 1 bis 22 für Endprodukte der allgemeinen Formel I, bei denen R² einen mit R³, R⁴ und R⁵ substituierten 2-Phenylethenylrest darstellt

Zur Herstellung der in den nachfolgenden Beispielen 1 bis 22 näher beschriebenen Verbindungen wurde folgendes Verfahren benutzt:

Verfahren C: Äquimoiare Mengen des entsprechend mit R¹ substituierten 4-Alkoxy-6-methyl-2H-pyran-2-ons der allgemeinen Formel III und des entsprechend mit R³, R⁴ und R⁵ substituierten Benzaldehyds der allgemeinen Formel IV werden in DMSO gelöst und bei 15 °C bis RT zunächst mit 0.1 Äquivalenten Tetraethylammoniumhydroxyd-Lösung (40 % in Wasser), dann (30 min bis 3 h später) mit 0.5 Äquivalenten Kaliumhydroxid (50 % in Wasser) versetzt und 15 bis 20 h unter Stickstoff bei RT gerührt. Das Reaktionsgemisch wird mit Wasser oder verdünnter Salzsäure verdünnt, das ausgefallene Produkt durch Filtration oder Extraktion mit Ethylacetat oder TBME gewonnen, über Kieselgel chromatographiert und / oder umkristallisiert.

| Beisp. Nr. | Name | Formel | Verf. | Ausg.-mat. Beisp. | Ausb. [%] | Schmp. [°C] (Lösungsmittel) |
|---|---|---|---|---|---|---|
| 1 | E-4-Ethoxy-6-[2-(3,4,5-trimethoxyphenyl)ethenyl]-2H-pyran-2-on | C18H20O6 | C | a) | 26 | 148 - 150 TBME/Ethanol |
| 2 | E-4-(1-Methylethoxy)-6-[2-(3,4,5-trimethoxyphenyl)ethenyl]-2H-pyran-2-on | C19H22O6 | C | 23 | 39 | 94 - 95 TBME/PE |
| 3 | E-4-Propoxy-6-[2-(3,4,5-trimethoxyphenyl)ethenyl]-2H-pyran-2-on | C19H22O6 | C | 24 | 28 | 117 - 118 TBME/Ethanol |
| 4 | E-4-(2-Methylpropoxy)-6-[2-(3,4,5-trimethoxyphenyl)ethenyl]-2H-pyran-2-on | C20H24O6 | C | 25 | 27 | 130 - 131 TBME/Ethanol |
| 5 | E-4-(Cyclopropylmethoxy)-6-[2-(3,4,5-trimethoxyphenyl)ethenyl]-2H-pyran-2-on | C20H22O6 | C | 27 | 39 | 148.5 - 149 TBME/Ethanol |
| 6 | E-4-(2-Methoxyethoxy)-6-[2-(3,4,5-trimethoxyphenyl)ethenyl]-2H-pyran-2-on | C19H22O7 | C | 28 | 34 | 145.5 - 146.5 PE/Ethanol |
| 7 | E-6-{2-[4-Ethoxy-3,5-dimethoxyphenyl]ethenyl}-4-(2-methoxyethoxy)-2H-pyran-2-on | C20H24O7 | C | 28 Ald # 172 | 33 | 132 - 133 PE/Ethylacetat |
| 8 | E-6-[2-(3,5-Dimethoxy-4-propoxyphenyl)ethenyl]-4-(2-methoxyethoxy)-2H-pyran-2-on | C21H26O7 | C | 28 Ald # 185 | 27 | 120 - 121 TBME |
| 9 | E-6-{2-[3,5-Dimethoxy-4-(2-methylpropoxy)phenyl]ethenyl}-4-(2-methoxyethoxy)-2H-pyran-2-on | C22H28O7 | C | 28 30 | 26 | 133 - 133.5 TBME/PE |
| 10 | E-6-{2-[4-Butoxy-3,5-dimethoxyphenyl]ethenyl}-4-(2-methoxyethoxy)-2H-pyran-2-on | C22H28O7 | C | 28 Ald # 193 | 25 | 123.5 - 124 TBME |
| 11 | E-6-[2-(4-Ethoxy-3,5-dimethoxyphenyl)ethenyl]-4-(1-methylethoxy)-2H-pyran-2-on | C20H24O6 | C | 23 Ald # 172 | 35 | 113.5 - 114 TBME/PE |
| 12 | E-4-(1-Methylethoxy)-6-[2-(3,5-dimethoxy-4-propoxyphenyl)-ethenyl]-2H-pyran-2-on | C21H26O6 | C | 23 Ald # 185 | 35 | 112 - 112.5 PE/Ethanol/TBME |
| 13 | (+-)-E-6-{2-[3,5-Dimethoxy-4-(1-methylpropoxy)phenyl]ethenyl}-4-(1-methylethoxy)-2H-pyran-2-on | C22H28O6 | C | 23 29 | 28 | 103 - 103.5 TBME/PE |
| 14 | 6-{2-[3,5-Dimethoxy-4-(2-methylpropoxy)phenyl]ethenyl}-4-(1-methylethoxy)-2H-pyran-2-on | C22H28O6 | C | 23 30 | 32 | 129.5 - 130.5 PE/TBME |
| 15 | E-6-[2-(4-Butoxy-3,5-dimethoxyphenyl)ethenyl]-4-(1-methylethoxy)-2H-pyran-2-on | C22H28O6 | C | 23 Ald # 193 | 40 | 93 - 94 PE/Isopropanol |
| 16 | E-6-[2-(3,5-Dimethoxy-4-pentyloxyphenyl)ethenyl]-4-(1-methylethoxy)-2H-pyran-2-on | C23H30O6 | C | 23 31 | 33 | 105.5 - 106.5 PE/Isopropanol |
| 17 | E-6-{2-[4-(Cyclopropylmethoxy)-3,5-dimethoxyphenyl]ethenyl}-4-(1-methylethoxy)-2H-pyran-2-on | C22H26O6 | C | 23 32 | 33 | 122.5 - 123 Ethanol/TBME/PE |
| 18 | E-6-{2-[4-(Cyclopentyloxy)-3,5-dimethoxyphenyl]ethenyl}-4-(1-methylethoxy)-2H-pyran-2-on | C23H28O6 | C | 23 33 | 33 | 107 - 107.5 Ethanol/TBME/PE |
| 19 | E-6-{2-[3,5-Dimethoxy-4-(2-propenyloxy)phenyl]ethenyl}-4-(1-methylethoxy)-2H-pyran-2-on | C21H24O6 | C | 23 Ald # 196 | 22 | 116 - 116.5 TBME/PE/Ethanol |
| 20 | E-6-{2-[3,5-Dimethoxy-4-(2-methoxyethoxy)phenyl]ethenyl}-4-(1-methylethoxy)-2H-pyran-2-on | C21H26O7 | C | 23 Ald # 203 | 40 | 88.5 - 89 TBME/PE |
| 21 | E-6-[2-(4-Ethoxy-3,5-dimethoxyphenyl)ethenyl]-4-propoxy-2H-pyran-2-on | C20H24O6 | C | 24 Ald # 172 | 19 | 132.5 - 133 TBME/PE/Ethanol |
| 22 | E-6-[2-(3-Ethoxy-4,5-dimethoxyphenyl)ethenyl]-4-(1-methylethoxy)-2H-pyran-2-on | C20H24O6 | C | 23 Ald # 243 | 29 | 120.5 - 121 TBME |

| | | | | | | |
|---|---|---|---|---|---|---|
| a) 6-Methyl-4-ethoxy-2H-pyran-2-on (bekannt: S. Sib Tetrahedron, 31, 2229-2235 (1975)) | | | | | | |

Die in den Beispielen 7, 8, 10, 11, 12, 15, 19, 20, 21 und 22 als Ausgangsmaterialien verwendeten Aldehyde sind die folgenden bekannten Verbindungen:
Ald. #172: 4-Ethoxy-3,5-dimethoxy-benzaldehyd
Ald. #185: 3,5-Dimethoxy-4-propoxy-benzaldehyd
Ald. #193: 4-Butoxy-3,5-dimethoxy-benzaldehyd
Ald. #196: 3,5-Dimethoxy-4-(2-propenyloxy)-benzaldehyd
Ald. #203: 3,5-Dimethoxy-4-(2-methoxyethoxy)-benzaldehyd
Ald. #243: 3-Ethoxy-4,5-dimethoxy-benzaldehyd

### II. Beispiele 23 bis 28 für Vorprodukte der allgemeinen Formel III

Zur Herstellung der in den nachfolgenden Beispielen 23 bis 28 näher beschriebenen Verbindungen wurden folgende Verfahren benutzt:

Verfahren D: 4-Hydroxy-6-methyl-2H-pyran-2-on, 1.5 Äquivalente des entsprechend substituierten Alkylbromids R¹Br, 1,5 Äquivalente Kaliumcarbonat und 0.2 Äquivalente Kaliumjodid werden in DMF 30 bis 40 h unter Stickstoff bei 80 °C gerührt. Man vernichtet überschüssiges Alkylierungsmittel mit Ammoniaklösung, filtriert, rotiert das Filtrat ein, nimmt in Ethylacetat auf, filtriert erneut oder wäscht mit Wasser und trocknet mit Natriumsulfat und rotiert ein. Der Rückstand wird umkristallisiert.

Verfahren E: Eine Lösung von 4-Hydroxy-6-methyl-2H-pyran-2-on in DMF wird bei 0 bis 15 °C zu einem Äquivalent Natriumhydrid (60 % in Weißöl) in DMF getropft und 1 bis 2 h bei 0 °C bis Raumtemperatur gerührt 1.1 bis 1.25 Äquivalente des entsprechend substituierten Alkylhalogenids R¹Cl, R¹Br bzw. R¹I in DMF und ggf. bis zu 0.1 Äquivalente Kaliumiodid werden zugegeben und 15 bis 20 h bei Raumtemperatur bis 100 °C unter Stickstoff gerührt. Überschüssiges Alkylierungsmittel wird gegebenenfalls mit Ammoniaklösung vernichtet. Das Reaktionsgemisch wird einrotiert, der Rückstand in Ethylacetat aufgenommen, gegebenenfalls mit Natronlauge und mit Wasser gewaschen, mit Natriumsulfat getrocknet und einrotiert. Das Rohprodukt wird umkristallisiert oder säulenchromatographisch gereinigt und gegebenenfalls im Vakuum destilliert.

| Beisp. Nr. | Name | Formel | Verf. | Ausb. [%] | Schmp. [°C] (Lösungsmittel) |
|---|---|---|---|---|---|
| 23 | 6-Methyt-4-(1-methylethoxy)-2H-pyran-2-on | C9H12O3 | D | 79 | 55 - 56 TBME/PE |
| 24 | 6-Methyl-4-propoxy-2H-pyran-2-on | C9H12O3 | E | 78 | Öl (Sdp. 100-102/0.03mbar) |
| 25 | 6-Methyl-4-(2-methylpropoxy)-2H-pyran-2-on | C10H14O3 | E | 47 | Öl |
| 26 | 4-Butyloxy-6-methyl-2H-pyran-2-on | C10H14O3 | E | 87 | Öl |
| 27 | 4-(Cyclopropylmethoxy)-6-methyl-2H-pyran-2-on | C10H12O3 | E | 62 | 70-70.5 PE/TBME |
| 28 | 4-(2-Methoxyethoxy)-6-methyl-2H-pyran-2-on | C9H12O4 | E | 63 | 77.5 - 78.5 TBME/PE |

### III. Beispiele 29 bis 33 für Vorprodukte der allgemeinen Formel IV

Zur Herstellung der in den nachfolgenden Beispielen 29 bis 33 näher beschriebenen Verbindungen wurde folgendes Verfahren benutzt:

Verfahren F: 4-Hydroxy-3,5-dimethoxybenzaldehyd, 2 Äquivalente des entsprechend substituierten Alkylhalogenids R⁴Cl, R⁴Br bzw. R⁴I, 1,5 bis 2 Äquivalente Kaliumcarbonat oder Kaliumhydroxid und gegebenenfalls 0.2 Äquivalente Kaliumjodid werden in DMF 15 bis 60 h unter Stickstoff bei 75 bis 90 °C gerührt. Man filtriert, rotiert das Filtrat ein, nimmt in Ethylacetat auf, wäscht mit Wasser, trocknet über Natriumsulfat und rotiert ein. Der Rückstand wird gegebenenfalls mittels Säulenchromatographie und / oder Umkristallisation gereinigt.

| Beisp. Nr. | Name | Formel | Verf. | Ausb. [%] | Schmp. [°C] (Lösungsmittel) |
|---|---|---|---|---|---|
| 29 | (+-)-3,5-Dimethoxy-4-(1-methylpropoxy)-benzaldehyd | C13H18O4 | F | 35 | Öl |
| 30 | 3,5-Dimethoxy-4-(2-methylpropoxy)-benzaldehyd | C13H18O4 | F | 58 | Öl |
| 31 | 3,5-Dimethoxy-4-pentyloxybenzaldehyd | C14H20O4 | F | 91 | ∼ 28 - 30 PE/TBME |
| 32 | 4-(Cyclopropylmethoxy)-3,5-dimethoxybenzaldehyd | C13H16O4 | F | 86 | 56.5 - 57 PE/TBME |
| 33 | 4-Cyclopentyloxy-3,5-dimethoxybenzaldehyd | C14H18O4 | F | 73 | 47.5 - 48 Isopropanol/Wasser |

### Pharmakologische Untersuchungen

Zur Ermittlung der anticonvulsiven / antiepileptischen Aktivität der erfindungsgemäßen Substanzen wurde die von E. A. Swinyard et al. (J. Pharmacol. Exp. Ther. 106, 319 (1952)) und von L. A. Woodbury et al. (Arch. int. Pharmacodyn. 92, 97 (1952) beschriebene Methodik verwendet. Männlichen Mäusen (NMRI) mit einem Körpergewicht von 20 - 25 g wird über Corneal-Elektroden ein Wechselstrom von 50 Hz und 50 mA während 0.2 s appliziert (HSE-Schock-Reizgerät, Typ 207). Der Maximal-Elektroschock-Krampf (MES) besteht aus einer tonischen Streckung der Hinterextremitäten, klonischen Zuckungen und einem Bewußtseinsverlust. Als Wirksamkeitskriterium wird die Hemmung des Extensorenkrampfes durch die erfindungsgemäßen Substanzen betrachtet Die Mäuse hatten vor den Experimenten freien Zugang zu Futter und Wasser. Die Testsubstanzen wurden als Suspension in 0.2 % Agar p. o. durch Schlundsonde oral appliziert; die Kontrolltiere erhielten adäquate Volumina Agar. 1 Stunde und 3 Stunden nach Applikation wurde der Test auf Schutzwirkung gegen MES durchgerührt.
In der nachstehenden Tabelle sind die Ergebnisse des MES-Tests bei Dosierungen von 100 mg der erfindungsgemäßen Verbindungen pro kg Körpergewicht aufgeführt. Als % Wirkung ist der prozentuale Anteil derjenigen Tiere angegeben, die bei dem MES-Test zu 100 % gegen den Extensorenkrampf geschützt waren.
Während der oben beschriebenen Experimente wurden die Tiere innerhalb der gesamten Versuchsdauer auf Anzeichen substanzbedingter Verhaltensänderungen und Neurotoxizität (Motilität, Muskeltonus, Atemfrequenz, Körpertemperatur und Allgemeinverhalten) beobachtet. Bei allen geprüften erfindungsgemäßen Substanzen konnten keine Nebenwirkungen festgestellt werden.

| Beisp. Nr. | Dosis [mg/kg] | Anzahl Tiere | Schutz nach 1 h [%] | Schutz nach 3 h [%] |
|---|---|---|---|---|
| 1 | 100 | 8 | 100 | 50 |
| 2 | 100 | 8 | 100 | 75 |
| 3 | 100 | 8 | 87.5 | 75 |
| 4 | 100 | 8 | 25 | 62.5 |
| 5 | 100 | 8 | 37.5 | 62.5 |
| 6 | 100 | 8 | 87.5 | 50 |
| 7 | 100 | 8 | 100 | 100 |
| 8 | 100 | 8 | 100 | 100 |
| 9 | 100 | 8 | 75 | 87.5 |
| 10 | 100 | 8 | 100 | 100 |
| 11 | 100 50 | 8 8 | 100 75 | 100 75 |
| 12 | 100 50 | 8 10 | 100 80 | 100 100 |
| 13 | 100 | 8 | 100 | 100 |
| 14 | 100 | 8 | 100 | 100 |
| 15 | 100 | 8 | 100 | 100 |
| 16 | 100 | 8 | 50 | 50 |
| 17 | 100 | 8 | 100 | 75 |
| 18 | 100 | 8 | 62.5 | 25 |
| 19 | 100 | 8 | 87.5 | 87.5 |
| 20 | 100 | 8 | 87.5 | 87.5 |
| 21 | 100 | 8 | 62.5 | 87.5 |
| 22 | 100 | 8 | 87.5 | 62.5 |

Nachfolgend die Daten von Kretzschmar und Meyer (Vergleich):

| | | | | |
|---|---|---|---|---|
| Methysticin | 70 | 10-45 | 80 | 17.5 |
| Dihydromethysticin | 70 | 10-45 | 55 | <10 |
| Kawain | 150 | 10-45 | 0 | 0 |
| Dihydrokawain | 150 | 10-45 | 0 | 0 |
| Yangonin | 750 | 10-45 | 20 | 40 |
| Desmethoxyyangonin | 400 | 10-45 | 50 (am Wirkungsmaximum nach 20 min) | |

### Beispiele für die Herstellung pharmazeutischer Zubereitungen der erfindungsgemäßen Substanzen:

### A. Tabletten:

Zur Herstellung von Tabletten, die je nach gewünschter Wirkungsstärke 5 bis 250 mg Wirkstoff enthalten, benötigt man

| | |
|---|---|
| erfindungsgemäße Substanz | 200 bis 5 000 g |
| Zellulosepulver | 2 000 g |
| Maisstärke | 1 200 g |
| kolloide Kieselsäure | 80 g |
| Magnesiumstearat | 20 g |
| Milchzucker | ad 10 000 g |

Der Wirkstoff wird gegebenenfalls gemahlen, mit den Hilfsstoffen homogen vermischt und in der üblichen Weise zu Tabletten von je 250 mg Gewicht und 9 mm Durchmesser verpreßt. Bei Dosierungen über 125 mg preßt man Tabletten von je 500 mg Gewicht und 11 mm Durchmesser. Falls gewünscht, werden die Tabletten mit einem Filmüberzug versehen.

### B. Kapseln:

Zur Herstellung von Kapseln, die je nach gewünschter Wirkungsstärke 5 bis 250 mg Wirkstoff enthalten, benötigt man

| | |
|---|---|
| erfindungsgemäße Substanz | 500 bis 12 500 g |
| Maisstärke | 2 000 g |
| kolloide Kieselsäure | 300 g |
| Magnesiumstearat | 50 g |
| Zellulosepulver | ad 20 000 g |

Die feingepulverten Stoffe werden homogen gemischt und in Hartgelatinekapseln der Größe 2 in der Menge von 200 mg pro Kapsel, oder bei Dosierungen über 125 mg in Hartgelatinekapseln der Größe 0 in der Menge von 400 mg pro Kapsel abgefüllt.

## Patentansprüche

1. 2H-1-Pyran-2-one der allgemeinen Formel I, 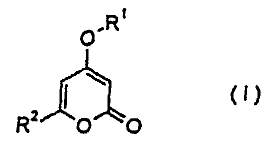 worin
R¹ einen Alkylrest mit 2 bis 5 C-Atomen, einen Cycloalkylrest mit 4 bis 6 C-Atomen, einen Cycloalkyl-alkylrest mit 4 bis 8 C-Atomen oder einen Alkoxyalkylrest mit insgesamt 3 bis 5 C-Atomen und
R² einen am Benzolring in den Positionen 3, 4 und 5 mit R³, R⁴ und R⁵ substituierten 2-Phenylethenylrest
darstellen, wobei unabhängig voneinander
R³ und R⁵ Methoxy oder Ethoxy und
R⁴ ein geradkettiger oder verzweigter Alkoxyrest mit 1 bis 5 C-Atomen, ein Alkenyloxyrest mit 3 bis 5 C-Atomen, ein Cycloalkoxyrest mit 4 bis 6 C-Atomen, ein Cycloalkylalkoxyrest mit 4 bis 8 C-Atomen oder einen Alkoxyalkoxyrest mit insgesamt 3 bis 5 C-Atomen sind.

2. Verbindungen der Formel I nach Anspruch 1, worin
R¹ einen Alkylrest mit 2 bis 5 C-Atomen, einen Cyclopropylmethylrest oder einen Methoxyethylrest und
R² einen am Benzolring in den Positionen 3, 4 und 5 mit R³, R⁴ und R⁵ substituierten 2-Phenylethenylrest
darstellen, wobei unabhängig voneinander
R³ und R⁵ Methoxy und
R⁴ ein geradkettiger oder verzweigter Alkoxyrest mit 1 bis 5 C-Atomen, ein Allyloxyrest, ein Cyclopentyloxyrest, ein Cyclopropylmethoxyrest oder ein Methoxyethoxyrest sind.

3. Verfahren zur Herstellung derjenigen Verbindungen der Formel I gemäß Anspruch 1 oder 2, worin R² ein am Benzolring in den Positionen 3, 4 und 5 mit R³, R⁴ und R⁵ substituierter 2-Phenylethenylrest ist, **dadurch gekennzeichnet, daß** ein Pyranon der Formel III 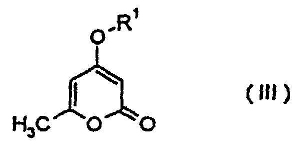 worin R¹ die in Anspruch 1 oder 2 genannte Bedeutung hat, mit einem in den Positionen 3, 4 und 5 mit R³, R⁴ und R⁵ substituierten Benzaldehyd der Formel IV 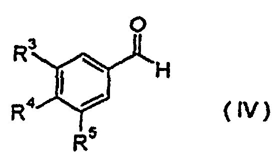 worin R³, R⁴ und R⁵ die in Anspruch 1 oder 2 genannte Bedeutung hat, kondensiert wird.

4. Reaktive Zwischenprodukte der allgemeinen Formel III zur Herstellung der Verbindungen gemäß Anspruch 1, 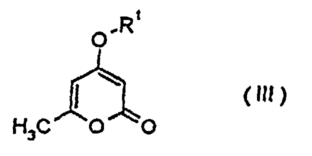 worin
R¹ einen Alkylrest mit 3 bis 5 C-Atomen, einen Cycloalkylrest mit 4 bis 6 C-Atomen, einen Cycloalkyl-alkylrest mit 4 bis 8 C-Atomen oder einen Alkoxyalkylrest mit insgesamt 3 bis 5 C-Atomen bedeutet

5. Pharmazeutische Zusammensetzung, enthaltend mindestens eine Verbindung gemäß einem der Ansprüche 1 oder 2, ggf. zusammen mit üblichen Hilfs- und Zusatzstoffen.

6. Verwendung der Verbindungen gemäß einem der Ansprüche 1 oder 2 zur Herstellung eines Arzneimittels mit antikonvulsiver und/oder antiepileptischer Wirkung.

7. Verwendung der Verbindungen gemäß einem der Ansprüche 1 oder 2 zur Herstellung eines Arzneimittels mit neuroprotektiver Wirkung und/oder mit Wirkung gegen neurodegenerative Erkrankungen bzw. Erscheinungen.

## Claims

1. 2H-1-pyran-2-ones of formula I, 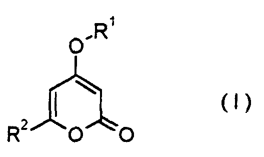 in which
**R**^{**1**} represents an alkyl radical with from 2 to 5 C atoms, a cycloalkyl radical with from 4 to 6 C atoms, a cycloalkyl alkyl radical with from 4 to 8 C atoms or an alkoxyalkyl radical with altogether 3 to 5 C atoms, and
**R**^{**2**} represents a 2-phenyl ethenyl radical substituted with **R**^{**3**}**, R**^{**4**} and **R**^{**5**} in positions 3, 4 and 5 on the benzene ring,
wherein, independently of one another,
**R**^{**3**} and **R**^{**5**} are methoxy or ethoxy, and
**R**^{**4**} is a straight-chain or branched alkoxy radical with from 1 to 5 C atoms, an alkenyloxy radical with from 3 to 5 C atoms, a cycloalkoxy radical with from 4 to 6 C atoms, a cycloalkyl alkoxy radical with from 4 to 8 C atoms or an alkoxy alkoxy radical with altogether 3 to 5 C atoms.

2. Compounds of the formula I according to claim 1, in which
**R**^{**1**} represents an alkyl radical with from 2 to 5 C atoms, a cyclopropylmethyl radical or a methoxyethyl radical, and
**R**^{**2**} represents a 2-phenyl ethenyl radical substituted with **R**^{**3**}**, R**^{**4**} and **R**^{**5**} in positions 3, 4 and 5 on the benzene ring,
wherein, independently of one another,
**R**^{**3**} and **R**^{**5**} are methoxy, and
**R**^{**4**} is a straight-chain or branched alkoxy radical with from 1 to 5 C atoms, an allyloxy radical, a cyclopentyloxy radical, a cyclopropyl methoxy radical or a methoxyethoxy radical.

3. A method of preparing the compounds of formula I according to Claim 1 or 2, in which **R**^{**2**} is a 2-phenyl ethenyl radical substituted with **R**^{**3**}**, R**^{**4**} and **R**^{**5**} in positions 3, 4 and 5 on the benzene ring, **characterized in that** a pyranone of formula III, 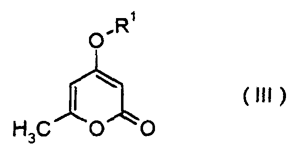 in which **R**^{**1**} has the meaning set out in Claim 1 or 2, is condensed with a benzaldehyde of formula **IV** 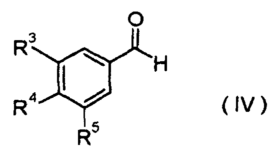 substituted with **R**^{**3**}**, R**^{**4**} and **R**^{**5**} in positions 3, 4 and 5, in which **R**^{**3**}**, R**^{**4**} and **R**^{**5**} have the meaning set out in Claim 1 or 2.

4. Reactive intermediate products of the general formula III for preparing the compounds according to Claim 1, 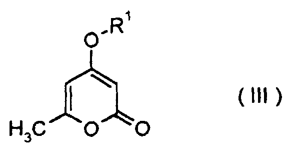 in which
**R**^{**1**} represents an alkyl radical with from 3 to 5 C atoms, a cycloalkyl radical with from 4 to 6 C atoms, a cycloalkyl alkyl radical with from 4 to 8 C atoms or an alkoxyalkyl radical with altogether 3 to 5 C atoms.

5. A pharmaceutical composition, containing at least one compound according to one of Claims 1 and 2, optionally together with conventional adjuvants and additives.

6. Use of the compounds according to one of Claims 1 and 2 for producing a medicament with an anti-convulsive and/or anti-epileptic effect.

7. Use of the compounds according to one of Claims 1 and 2 for producing a medicament with a neuro-protective effect and/or with an effect against neuro-degenerative diseases or symptoms.

## Revendications

1. 2H-1-pyran-2-ones de la formule générale I, 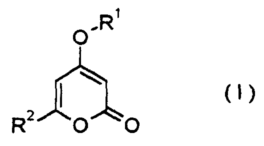 dans laquelle
**R**^{**1**} représente un groupement alkyle avec 2 à 5 Atomes de C, un groupement cycloalkyle avec 4 à 6 atomes de C, un groupement cycloalkyle-alkyle avec 4 à 8 atomes de C ou un groupement alcoxyalkyle avec, ensemble, 3 à 5 atomes de C, et
**R**^{**2**} représente un groupement 2-phényléthényle substitué sur le cycle benzénique aux positions 3, 4, et 5 par **R**^{**3**}**, R**^{**4**} et **R**^{**5**}**,**
et, indépendamment les uns des autres, les groupements
**R**^{**3**} et **R**^{**5**} étant méthoxyle ou éthoxyle, et
**R**^{**4**} étant un groupement alcoxy avec 1 à 5 atomes de C à chaîne droite ou ramifiée, un groupement alkényloxy avec 3 à 5 atomes de C, un groupement cycloalcoxy avec 4 à 6 atomes de C, un groupement cycloalkylalcoxy avec 4 à 8 atomes de C ou un groupement alcoxyalcoxy avec, ensemble, 3 à 5 atomes de C.

2. Composés de la formule I selon la revendication 1, dans laquelle
**R**^{**1**} représente un groupement alkyle avec 2 à 5 Atomes de C, un groupement cyclopropylméthyle ou un groupement méthoxyéthyle, et
**R**^{**2**} représente un groupement 2-phényléthényle substitué sur le cycle benzénique aux positions 3, 4, et 5 par **R**^{**3**}**, R**^{**4**} et **R**^{**5**}**,**
les groupements étant indépendants les uns des autres
**R**^{**3**} et **R**^{**5**} étant méthoxyle, et
**R**^{**4**} étant un groupement alcoxy avec 1 à 5 atomes de C à chaîne droite ou ramifiée, un groupement allyloxy, un groupement cyclopentyloxy, un groupement cyclopropylméthoxy ou un groupement méthoxy-éthoxy.

3. Procédé pour la fabrication desdits produits de la formule I selon la revendication 1 ou 2, dans laquelle **R**^{**2**} est un groupement 2-phényléthényle substitué sur le cycle benzénique aux positions 3, 4, et 5 par **R**^{**3**}**, R**^{**4**} et **R**^{**5**}**, caractérisé en ce qu'**un pyranone de la formule III, 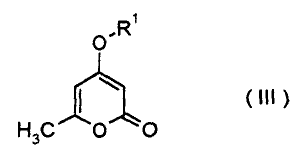 dans laquelle **R**^{**1**} a la signification connue énoncée dans la revendication 1 ou 2, est condensé avec un benzaldéhyde substitué aux positions 3, 4 et 5 par **R**^{**3**}**, R**^{**4**} et **R**^{**5**} de la formule IV 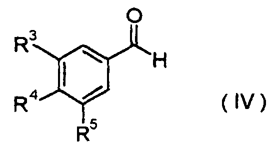 dans laquelle **R**^{**3**}**, R**^{**4**} et **R**^{**5**} ont la signification énoncée dans la revendication 1 ou 2.

4. Produits intermédiaires réactifs de la formule générale III pour la fabrication des composés selon la revendication 1, 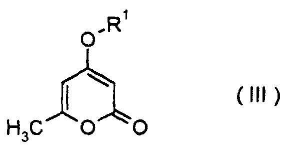 dans laquelle
**R**^{**1**} représente un groupement alkyle avec 3 à 5 atomes de C, un groupement cycloalkyle avec 4 à 6 atomes de C, un groupement cycloalkyle-alkyle avec 4 à 8 atomes de C ou un groupement alcoxyalkyle avec, ensemble, 3 à 5 atomes de C.

5. Composition pharmaceutique, comprenant au moins un composé selon une des revendications 1 ou 2, le cas échéant associé avec des agents auxiliaires et des agents d'addition usuels.

6. Utilisation des composés selon une des revendications 1 ou 2 pour la fabrication d'un médicament avec un effet anticonvulsif et/ou antiépileptique.

7. Utilisation des composés selon une des revendications 1 ou 2 pour la fabrication d'un médicament avec un effet neuroprotecteur et/ou un effet contre les maladies ou bien les manifestations neurodégénératives.
